# EUROPEAN PATENT SPECIFICATION

(11) **EP 4 435 917 B1**
(45) Date of publication and mention of the grant of the patent: **06.05.2026**
(21) Application number: 24156294.1
(22) Date of filing: 07.02.2024
(51) Int. Cl.: H01M 10/0525, H01M 10/0567

(54) **ELECTROLYTE FOR RECHARGEABLE LITHIUM BATTERY AND RECHARGEABLE LITHIUM BATTERY INCLUDING THE SAME**
ELEKTROLYT FÜR EINE WIEDERAUFLADBARE LITHIUMBATTERIE UND WIEDERAUFLADBARE LITHIUMBATTERIE DAMIT
ÉLECTROLYTE POUR BATTERIE AU LITHIUM RECHARGEABLE ET BATTERIE AU LITHIUM RECHARGEABLE LE COMPRENANT

(30) Priority: 24.03.2023 KR 20230038844
(43) Date of publication of application: 25.09.2024
(73) Proprietor: Samsung SDI Co., Ltd., Yongin-si, Gyeonggi-do 17084 (KR)
(72) Inventor: Bae, Tae Hyon, 17084 Yongin-si, Gyeonggi-do (KR); Lee, Harim, 17084 Yongin-si, Gyeonggi-do (KR); Kim, Sanghyung, 17084 Yongin-si, Gyeonggi-do (KR); Son, Seunghyeon, 17084 Yongin-si, Gyeonggi-do (KR); Yu, Arum, 17084 Yongin-si, Gyeonggi-do (KR); Kim, Yunhee, 17084 Yongin-si, Gyeonggi-do (KR)
(74) Representative: Gulde & Partner

(56) References cited:
- CN-B- 108 373 142
- DING FEI ET AL: "Effects of Cesium Cations in Lithium Deposition via Self-Healing Electrostatic Shield Mechanism", THE JOURNAL OF PHYSICAL CHEMISTRY C, vol. 118, no. 8, 14 February 2014 (2014-02-14), US, pages 4043 - 4049, XP093191504, ISSN: 1932-7447, DOI: 10.1021/jp4127754

## Description

### BACKGROUND

### 1. Field

One or more aspects of embodiments of the present disclosure relate to an electrolyte for a rechargeable lithium battery and a rechargeable lithium battery including the electrolyte.

### 2. Description of the Related Art

A rechargeable lithium battery may be recharged and has three or more times as high energy density per unit weight as a comparable lead storage battery, nickel-cadmium battery, nickel hydrogen battery, nickel zinc battery and/or the like. It may be also charged at a relatively high rate and thus, is commercially manufactured for a laptop, a cell phone, an electric tool, an electric bike, and/or the like, and research on improvement of additional energy density have been actively made.

Such a rechargeable lithium battery is manufactured by injecting an electrolyte into a battery cell, which includes a positive electrode including a positive electrode active material capable of intercalating/deintercalating lithium ions and a negative electrode including a negative electrode active material capable of intercalating/deintercalating lithium ions. An organic solvent in which a lithium salt is dissolved is utilized as an electrolyte, and the electrolyte is important in determining stability and performance of a rechargeable lithium battery.

Implementation of a high-capacity and high-energy density battery requires (or there is a desire for) the design of a battery drivable at a relatively high voltage of 4.5 V or greater, that should result in increased energy-density and/or electrode active material density of the electrode, and improved high-speed charging performance. However, under severe conditions such as in the high voltage and the high-speed charging, deterioration of the positive electrode and growth of lithium dendrites on the surface of the negative electrode accelerate undesired reaction(s) (e.g., undesired side reaction(s)) between the electrodes and the electrolyte. The undesired reaction(s) may reduce the cycle-life of the battery and initiate gas generation and/or the like that may compromise the range of conditions for safe operation of the battery (e.g., battery safety may be comprised or aggravated due to gas generation, and/or the like).

Methods of protecting the electrodes through surface treatment to reduce or suppress the undesired reaction(s) with the electrolyte have been investigated. However, studies of surface treatment of the positive electrode have not suitably achieved and/or have failed to achieve sufficient protection effects under high voltage-driving conditions, and the surface treatment of the negative electrode has been reported to cause an additional problem of deteriorating battery capacity. Accordingly, in the design of high-capacity electrodes capable of operating at relatively a relatively high voltage with relatively high-speed charging, development of an electrolyte capable of improving battery safety and performance is desired, needed, and/or required.

Current techniques for applying a low viscosity ester-based solvent to the electrolyte to suppress or reduce lithium dendrites of the negative electrode and thus improve electrolyte impregnability have been investigated. However, the ester-based solvent has relatively low oxidation resistance and relatively high flammability which may disadvantageously deteriorate battery performance and safety at high voltage.

### SUMMARY

The present invention is set out in the appended set of claims, wherein the drawings and respective description relate to advantageous embodiments thereof. It was an object of the present invention to provide an electrolyte capable of reducing or suppressing generation of lithium dendrites in the negative electrode by improving the impregnation of the negative electrode (e.g., by improving conduction of lithium ions of the negative electrode) and uniformizing the intercalation of lithium ions on the surface of the negative electrode.

It was also an object of the present invention to provide a rechargeable lithium battery with an improved charging performance, high-temperature cycle-life characteristics, and safety.

In some embodiments, an electrolyte for a rechargeable lithium battery includes a non-aqueous organic solvent, a lithium salt, and an additive, wherein the additive includes a first compound selected from among a compound represented by Chemical Formula 1, CsPF₆, and a combination thereof; and an Ag salt.

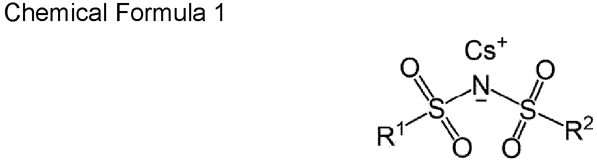

In Chemical Formula 1, R¹ and R² may each independently be a fluoro group or a substituted C1 to C10 fluoroalkyl group containing at least one fluorine (F).

R¹ and R² in Chemical Formula 1 may each independently be a fluoro group or a C1 to C4 fluoroalkyl group substituted with at least three fluoro groups.

Chemical Formula 1 may be represented by any one selected from among Chemical Formula 1-1 and Chemical Formula 1-2.

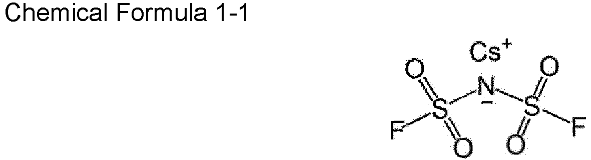

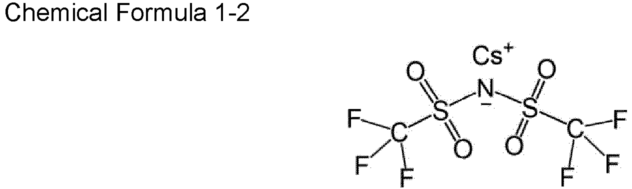

The first compound may be included (e.g., be present) in an amount of 0.1 wt% to 5.0 wt% based on a total weight of the electrolyte for a rechargeable lithium battery.

The Ag salt may include at least one selected from AgNO₃, AgPF₆, AgFSI, AgTFSI, AgF, AgSO₃CF₃, AgBF₄, AgNO₂, AgN₃, and AgCN.

The Ag salt may be included (e.g., be present) in an amount of 0.1 wt% to 10.0 wt% based on a total weight of the electrolyte for a rechargeable lithium battery.

The first compound and the Ag salt may be included (e.g., be present) in a weight ratio of 1:9 6:4.

The additive may further include a fluorinated ketone.

The fluorinated ketone may be represented by Chemical Formula 2.

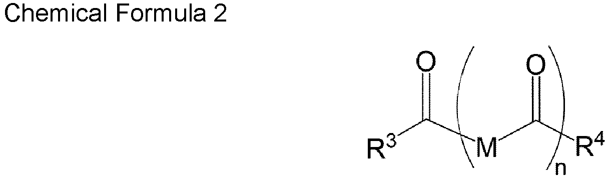

In Chemical Formula 2,
M is a substituted or unsubstituted C1 to C30 alkylene group, a substituted or unsubstituted C2 to C30 alkenylene group, or a substituted or unsubstituted C2 to C30 alkynylene group,
n is one of the integers from 0 to 5, and
R³ and R⁴ may each independently be a substituted or unsubstituted C1 to C30 alkyl group, a substituted or unsubstituted C2 to C30 alkenyl group, a substituted or unsubstituted C2 to C30 alkynyl group, a substituted or unsubstituted C3 to C20 cycloalkyl group, or a substituted or unsubstituted C6 to C20 aryl group, wherein at least one of R³ or R⁴ may include one or more fluorine(s).

At least one of R³ or R⁴ in Chemical Formula 2 may be a C1 to C10 fluoroalkyl group in which at least one hydrogen is substituted with fluorine.

At least one of R³ or R⁴ in Chemical Formula 2 may be a C1 to C10 fluoroalkyl group substituted with 3 to 21 fluorines.

At least one of R³ or R⁴ in Chemical Formula 2 may be a C1 to C10 perfluoroalkyl group in which all hydrogens are substituted with fluorine.

The fluorinated ketone is represented by Chemical Formula 2-1.

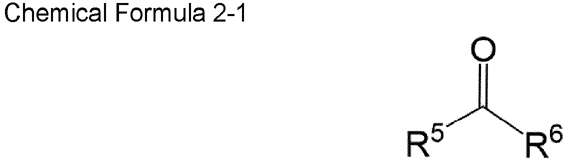

In Chemical Formula 2-1,
R⁵ and R⁶ may each independently be, a substituted or unsubstituted C1 to C30 alkyl group, and at least one of R⁵ or R⁶ includes one or more fluorine(s).

At least one of R⁵ or R⁶ in Chemical Formula 2-1 may be a C1 to C10 perfluoroalkyl group in which all hydrogens are substituted with fluorine.

R⁵ and R⁶ in Chemical Formula 2-1 may be different from each other, and Chemical Formula 2-1 has an asymmetric structure.

The fluorinated ketone may be represented by one of Chemical Formula 2-2 and Chemical Formula 2-3.

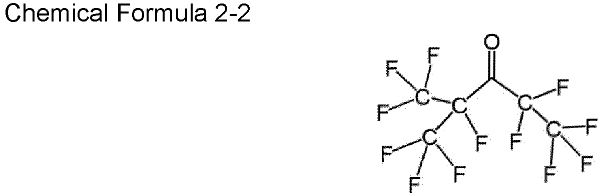

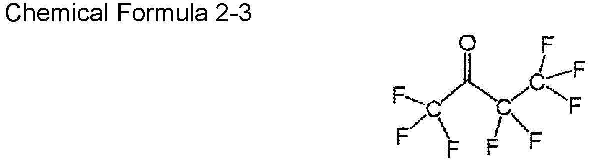

The fluorinated ketone may be included (e.g., be present) in an amount of 1.0 wt% to 10.0 wt% based on a total weight of the electrolyte for a rechargeable lithium battery.

The fluorinated ketone and the Ag salt may be included (e.g., be present) in a weight ratio of 1:9 to 6:4.

The additive may be included (e.g., be present) in an amount of 1.0 wt% to 30.0 wt% based on a total weight of the electrolyte for a rechargeable lithium battery.

The electrolyte for a rechargeable lithium battery further includes other additives, and
the other additives may include at least one selected from among vinylene carbonate (VC), fluoroethylene carbonate (FEC), difluoroethylene carbonate (DFEC), chloroethylene carbonate (CEC), dichloroethylene carbonate (DCEC), bromoethylene carbonate (BEC), dibromoethylene carbonate (DBEC), nitroethylene carbonate, cyanoethylene carbonate, vinylethylene carbonate (VEC), succinonitrile (SN), adiponitrile (AN), 1,3,6-hexane tricyanide (HTCN), propene sultone (PST), propane sultone (PS), lithium tetrafluoroborate (LiBF₄), lithium difluorophosphate (LiPO₂F₂), 2-fluoro biphenyl (2-FBP), lithium difluorooxalatoborate (LiDFOB), and lithium bisoxalatoborate (LiBOB).

According to some embodiments, a rechargeable lithium battery includes a positive electrode including a positive electrode active material, a negative electrode including a negative electrode active material, a separator between the positive electrode and the negative electrode, and the aforementioned electrolyte for a rechargeable lithium battery.

The negative electrode may have a negative electrode active material density (e.g., the density of the negative active material in the electrode) of greater than or equal to 1.6 gram per cubic centimeter (g/cc).

The electrolyte for a rechargeable lithium battery according to some embodiments can improve the impregnability (e.g., the impregnability of the lithium ions) of the negative electrode and can uniformly insert lithium ions into the surface of the negative electrode, thereby suppressing generation of lithium dendrites in the negative electrode.

The rechargeable lithium battery according to some embodiments includes the electrolyte for a rechargeable lithium battery, so that high-speed charging performance, high-temperature cycle-life characteristics, and safety may be improved.

### BRIEF DESCRIPTION OF THE DRAWINGS

FIG. 1 is a schematic view illustrating a rechargeable lithium battery according to some embodiments of the present disclosure.
FIG. 2 is a graph showing discharge capacity retention rates of rechargeable lithium battery cells according to evaluation of high-temperature cycle-life characteristics of the present disclosure.

### DETAILED DESCRIPTION

Hereinafter, example embodiments of the present disclosure will be described in more detail so that a person skilled in the art would understand the same. However, this disclosure may be embodied in many different forms and is not to be construed as limited to the embodiments set forth herein. In this regard, the present embodiments may have different forms and should not be construed as being limited to the descriptions set forth herein. Accordingly, the embodiments are merely described, by referring to the drawings, to explain aspects of the present description. As utilized herein, the term "and/or" includes any and all combinations of one or more of the associated listed items. Expressions such as "at least one of," if (e.g., when) preceding a list of elements, modify the entire list of elements and do not modify the individual elements of the list.

In the present specification It will be understood that if (e.g., when) an element such as a layer, film, region, or substrate is referred to as being "on" another element, it may be directly on the other element or intervening elements may also be present. In contrast, if (e.g., when) an element is referred to as being "directly on" another element, there are no intervening elements present.

In some embodiments, "layer" herein includes not only a shape formed on the whole surface if (e.g., when) viewed from a plan view, but also a shape formed on a partial surface.

As used herein, expressions such as "at least one of," "one of," and "selected from among," if (e.g., when) preceding a list of elements, modify the entire list of elements and do not modify the individual elements of the list. As utilized herein, the expressions "at least one of A, B, or C", "one of A, B, C, or a combination thereof" and "one of A, B, C, and a combination thereof" refer to each component and a combination thereof (e.g., A; B; A and B; A and C; B and C; or A, B, and C). For example, "at least one of a to c," "at least one of a, b or c," and "at least one of a, b and/or c" may indicate only a, only b, only c, both (e.g., simultaneously) a and b, both (e.g., simultaneously) a and c, both (e.g., simultaneously) b and c, all of a, b, and c, or variations thereof.

Herein, "or" is not to be construed as an exclusive meaning, for example, "A or B" is construed to include A, B, A+B, and/or the like.

Hereinafter, as the present disclosure allows for one or more suitable changes and numerous embodiments, specific embodiments will be illustrated in the drawings and described in more detail in the written description. However, this is not intended to limit the present disclosure to particular modes of practise.

The terms utilized herein are merely utilized to describe specific embodiments and are not intended to limit the present disclosure. Unless otherwise defined, all chemical names, technical and scientific terms, and terms defined in common dictionaries should be interpreted as having meanings consistent with the context of the related art, and should not be interpreted in an ideal or overly formal sense. It will be understood that, although the terms first, second, etc. may be used herein to describe various elements, these elements should not be limited by these terms. These terms are only used to distinguish one element from another element. Thus, a first element could be termed a second element without departing from the teachings of the present disclosure. Similarly, a second element could be termed a first element.

As utilized herein, it is to be understood that the terms such as "including," "includes," "include," "having," "has," "have," "comprises," "comprise," and/or "comprising" are intended to indicate the existence of the features, numbers, steps, actions, components, parts, ingredients, materials, or combinations thereof disclosed in the specification and are not intended to preclude the possibility that one or more other features, numbers, steps, actions, components, parts, ingredients, materials, or combinations thereof may exist or may be added. The symbol "/"utilized herein may be interpreted as "and" or "or" according to the context. The term "combination thereof may include a mixture, a laminate, a complex, a copolymer, an alloy, a blend, a reactant of constituents.

As used herein, singular forms such as "a," "an," and "the" are intended to include the plural forms as well, unless the context clearly indicates otherwise.

As used herein, the terms "use," "using," and "used" may be considered synonymous with the terms "utilize," "utilizing," and "utilized," respectively.

As used herein, the term "and/or" includes any and all combinations of one or more of the associated listed items.

The term "may" will be understood to refer to "one or more embodiments of the present disclosure," some of which include the described element and some of which exclude that element and/or include an alternate element. Similarly, alternative language such as "or" refers to "one or more embodiments of the present disclosure," each including a corresponding listed item.

In the drawings, the thicknesses of layers and regions are exaggerated or reduced for clarity. Like reference numerals in the drawings denote like elements throughout, and duplicative descriptions thereof may not be provided the specification. Throughout the specification, while such terms as "first," "second," and/or the like may be utilized to describe one or more suitable components, such components should not be limited to the above terms. The above terms are utilized only to distinguish one component from another. Components having substantially the same functional configuration in the present specification and drawings are denoted by the same reference numerals, and redundant descriptions thereof will not be provided.

Spatially relative terms, such as "beneath," "below," "lower," "above," "upper," "bottom," "top," and the like, may be used herein for ease of description to describe one element or feature's relationship to another element(s) or feature(s) as illustrated in the drawings. It will be understood that the spatially relative terms are intended to encompass different orientations of the device in use or operation in addition to the orientation depicted in the drawings. For example, if the device in the drawings is turned over, elements described as "below" or "beneath" other elements or features would then be oriented "above" or "over" the other elements or features. Thus, the term "below" may encompass both an orientation of above and below. The device may be otherwise oriented (rotated 90 degrees or at other orientations), and the spatially relative descriptors used herein should be interpreted accordingly.

In this context, "consisting essentially of" means that any additional components will not materially affect the chemical, physical, optical or electrical properties of the semiconductor film.

Further, in this specification, the phrase "on a plane," or "plan view," means viewing a target portion from the top, and the phrase "on a cross-section" means viewing a cross-section formed by vertically cutting a target portion from the side.

### Definitions

Hereinafter, the term "combination" includes a mixture of two or more, a composite, a copolymer, an alloy, a blend, mutual substitution, and a stacked structure of two or more.

As utilized herein, if (e.g., when) a definition is not otherwise provided, "substituted" refers to replacement of hydrogen of a substituent or a compound by deuterium, a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 fluoroalkyl group, a cyano group, or a combination thereof. For example, "substituted" refers to replacement of at least one hydrogen of a substituent or a compound by deuterium, a cyano group, a halogen, a methyl group, an ethyl group, a propyl group, a butyl group, a phenyl group, a biphenyl group, a terphenyl group, a trifluoromethyl group, or a naphthyl group, for example, a fluoro group, a chloro group, a bromo group, an iodo group, a methyl group, an ethyl group, a propyl group, or a butyl group, for example, a fluoro group, a chloro group, a bromo group, or an iodo group.

Herein, expressions such as C1 to C30 refer to that the number of carbons is 1 to 30.

In some embodiments, the average particle diameter may be measured by a method well suitable to those skilled in the art, for example, may be measured by a particle size analyzer, or may be measured by a transmission electron microscopic image or a scanning electron microscopic image. In some embodiments, it is possible to obtain an average particle diameter value by measuring utilizing a dynamic light scattering method, performing data analysis, counting the number of particles for each particle size range, and calculating from this. As utilized herein, if (e.g., when) a definition is not otherwise provided, the average particle diameter may refer to a diameter (D50) of particles having a cumulative volume of 50 vol% in a particle size distribution.

Hereinafter, a more detailed configuration of a rechargeable lithium battery according to some embodiments will be described.

### Electrolyte

An electrolyte for a rechargeable lithium battery according to some embodiments includes a non-aqueous organic solvent, a lithium salt, and an additive. In some embodiments, the additive includes a first compound selected from among a compound represented by Chemical Formula 1, CsPF₆, and a combination thereof; and an Ag salt. In some embodiments, the additive includes a first compound that is a compound represented by Chemical Formula 1, CsPF₆, or a combination thereof, and the additive includes an Ag salt.

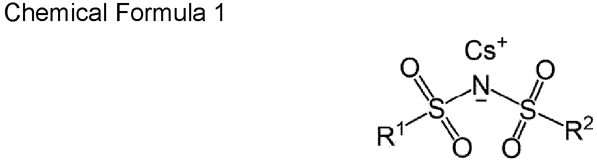

In Chemical Formula 1, R¹ and R² may each independently be a fluoro group or a C1 to C10 fluoroalkyl group substituted with at least one fluorine.

In one embodiment, the additive comprises as a first compound a compound represented by Chemical Formula 1 and an Ag salt. In an alternative embodiment, the additive comprises as a first compound CsPF₆ and an Ag salt. In another embodiment, the additive comprises as a first compound a combination of a compound represented by Chemical Formula 1 and CsPF₆ and an Ag salt. In a preferred embodiment the additive comprises as a first compound a compound represented by Chemical Formula 1 and an Ag salt.

Because the electrolyte for a rechargeable lithium battery according to some embodiments includes the compound represented by Chemical Formula 1 or CsPF₆, the compound is decomposed in the electrolyte and forms a film on the surfaces of the positive and negative electrodes, effectively controlling elution of lithium ions generated from the positive electrode and thus preventing or reducing the positive electrode decomposition phenomenon. For example, the compound represented by Chemical Formula 1 is earlier (e.g., more easily) reduced and decomposed compared to a carbonate-based solvent included in the non-aqueous organic solvent. The compound represented by Chemical Formula 1 forms an SEI (solid electrolyte interface) film on the negative electrode that may prevent or reduce decomposition of the electrolyte and the associated decomposition of the electrode, and may reduce or suppress an increase in internal resistance due to gas generation. The SEI film formed on the negative electrode is partially decomposed through a reduction reaction during the charge and discharge of the battery, and moves toward the positive electrode surface to form a film on the positive electrode surface through an oxidation reaction. This film may prevent or reduce decomposition of the positive electrode surface, and any oxidation reactions of the electrolyte, thereby contributing to the improvement of high-temperature and low-temperature cycle-life characteristics.

In other words, the electrolyte includes the compound represented by Chemical Formula 1 or CsPF₆ and thus may improve cycle-life characteristics and safety of the battery (e.g., the range of safe operating conditions).

In some embodiments, because the Ag salt improves conductivity (e.g., impregnability) of the lithium ions in the rechargeable lithium battery, the lithium ions may effectively move at (e.g., in) a negative electrode having high density (e.g., high active material density) and thus suppress or reduce generation of lithium dendrites. Accordingly, the side reaction between the electrolyte including the above and the electrodes may be reduced, improving high-temperature cycle-life characteristics and safety of the rechargeable lithium battery.

### First Compound

The first compound includes only one or both (e.g., simultaneously) selected from among the compound represented by Chemical Formula 1 and CsPF₆ (for example, meaning that the two compounds are mixed).

In Chemical Formula 1, R¹ and R² may each independently be a fluoro group or a substituted C1 to C4 fluoroalkyl group including at least one fluorine.

For example, R¹ and R² in Chemical Formula 1 may each independently be a fluoro group or a C1 to C4 fluoroalkyl group substituted with at least three fluoro groups.

For example, R¹ and R² in Chemical Formula 1 may each independently be a fluoro group or a C1 to C3 fluoroalkyl group substituted with at least three fluoro groups.

For example, R¹ and R² in Chemical Formula 1 may each independently be a fluoro group or a C1 to C2 fluoroalkyl group substituted with at least three fluoro groups.

In Chemical Formula 1, R¹ and R² may be the same as or different from each other.

As an example of a specific chemical formula, it may be represented by any one of Chemical Formula 1-1 and Chemical Formula 1-2.

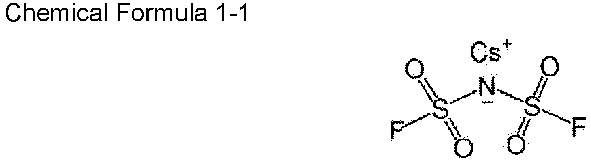

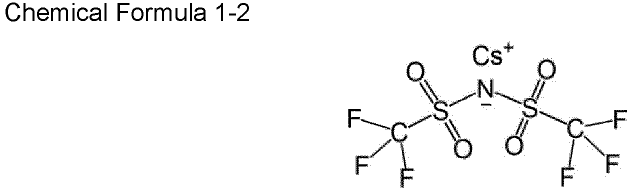

The first compound may be included in an amount of 0.1 wt% to about 5.0 wt%, 0.2 wt% to 4 wt%, 0.25 wt% to 3 wt%, 0.35 wt% to 2 wt% and 0.3 wt% to 1 wt%, based on a total weight of the electrolyte for a rechargeable lithium battery. For example, it may be included in an amount of greater than or equal to 0.10 wt%, for example greater than or equal to 0.20 wt%, for example greater than or equal to 0.25 wt%, for example greater than or equal to 0.30 wt%, or for example greater than or equal to 0.35 wt%, and for example less than or equal to 5.0 wt%, for example less than or equal to 4.0 wt%, for example less than or equal to 3.0 wt%, for example less than or equal to 2.0 wt%, or for example less than or equal to 1.0 wt%.

By including the first compound within the above range in the electrolyte according to some embodiments, an increase in internal resistance of the battery due to decomposition of the electrolyte may be suppressed or reduced, and cycle-life characteristics and safety of the battery may be improved.

### Ag Salt

The Ag salt included in the electrolyte for a rechargeable lithium battery according to the present invention refers to a compound including an Ag cation and an anion paired therewith, and the type or kind of anion may include nitrate ion, carbonate ion, sulfate ion, chloride ion, or sulfide ion.

The Ag salt may include, for example, one or more selected from AgNO₃, AgPF₆, AgFSI, AgTFSI, AgF, AgSO₃CF₃, AgBF₄, AgNO₂, AgN₃, and AgCN. For example, the Ag salt may be silver nitrate, silver carbonate, or silver sulfate, for example, silver nitrate. In the case of the silver nitrate, it can be effectively dissolved in an electrolyte, and is advantageous in suppressing lithium dendrites generated in a high-density negative electrode by improving lithium ion conductivity in the negative electrode. However, it is not limited to silver nitrate, and any compound that is soluble in an electrolyte and contains Ag cations can be utilized as the Ag salt.

The Ag salt may be included in an amount of about 0.1 wt% to 10.0 wt%, 0.2 wt% to 9.0 wt%, 0.3 wt% to 8.5 wt%, 0.4 wt% to 8.0 wt%, 0.5 wt% to 7.5 wt%, 1.0 wt% to 7.0 wt%, 2.0 wt% to 6.5 wt% or 3.0 wt% to 6.0 wt%, based on a total weight of the electrolyte for a rechargeable lithium battery. For example, it may be included in an amount of greater than or equal to 0.10 wt%, for example greater than or equal to 0.20 wt%, for example greater than or equal to 0.30 wt%, for example greater than or equal to 0.40 wt%, for example greater than or equal to 0.50 wt%, for example greater than or equal to 1.0 wt%, for example greater than or equal to 2.0 wt%, or for example greater than or equal 3.0 wt%, and for example less than or equal to 10.0 wt%, for example less than or equal to 9.0 wt%, for example less than or equal to 8.5 wt%, for example less than or equal to 8.0 wt%, for example less than or equal to 7.5 wt%, or for example less than or equal to 7.0 wt%, but is not limited thereto. By including the Ag salt within the above ranges in the electrolyte according to some embodiments, generation of dendrite in a battery negative electrode including the Ag salt may be suppressed or reduced.

A weight ratio of the first compound and the Ag salt may be 1:9 to 6:4, for example, 1:1, for example, 1:2, for example, 1:3, for example, 1:4, but is not limited thereto.

### Fluorinated Ketone

The electrolyte for a rechargeable lithium battery according to some embodiments may further include a fluorinated ketone. The fluorinated ketone is a compound having a ketone group, and may be a ketone compound including fluorine by replacing at least one hydrogen with fluorine.

By further including a fluorinated ketone, the electrolyte for a rechargeable lithium battery may have flame retardant properties and more effectively suppress or reduce lithium dendrites generated in a negative electrode, and a rechargeable lithium battery utilizing such an electrolyte may have improved safety and battery characteristics. In particular, the overall performance of a battery to which a high-density negative electrode of greater than or equal to 1.6 g/cc in active material and a high voltage of 4.5 V or greater are applied may be improved, and high-speed charging performance of 3.0 C level may be improved.

For example, because the fluorinated ketone has a low viscosity, it is possible to improve the negative electrode impregnability of an electrolyte including the fluorinated ketone. In some embodiments, the fluorinated ketone may have flame retardant properties by including at least one fluorine in the compound. Accordingly, the rechargeable lithium battery including the same may improve safety.

The fluorinated ketone may be represented by Chemical Formula 2.

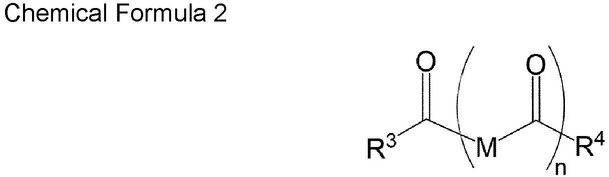

In Chemical Formula 2, M is a substituted or unsubstituted C1 to C30 alkylene group, a substituted or unsubstituted C2 to C30 alkenylene group, or a substituted or unsubstituted C2 to C30 alkynylene group, n is one of the integers from 0 to 5, and R³ and R⁴ may each independently be, a substituted or unsubstituted C1 to C30 alkyl group, a substituted or unsubstituted C2 to C30 alkenyl group, a substituted or unsubstituted C2 to C30 alkynyl group, a substituted or unsubstituted C3 to C20 cycloalkyl group, or a substituted or unsubstituted C6 to C20 aryl group, wherein at least one of R³ or R⁴ may include one or more fluorines.

In Chemical Formula 2, M may be, for example, a substituted or unsubstituted C1 to C30 alkylene group, for example, a substituted or unsubstituted C1 to C20 alkylene group, for example, a substituted or unsubstituted C1 to C10 alkylene group, for example, a substituted or unsubstituted C1 to C5 alkylene group, for example, a substituted or unsubstituted C1 to C4 alkylene group, for example, a substituted or unsubstituted C1 to C3 alkylene group. For example, M may be a C1 to C10 fluoroalkylene group in which one or more hydrogen atoms are substituted with fluorine.

In Chemical Formula 2, R³ and R⁴ may each independently be a substituted or unsubstituted C1 to C10 alkyl group, a substituted or unsubstituted C2 to C10 alkenyl group, or a substituted or unsubstituted C2 to C10 alkynyl group. For example, it may be a substituted or unsubstituted C1 to C5 alkyl group, a substituted or unsubstituted C2 to C5 alkenyl group, or a substituted or unsubstituted C2 to C5 alkynyl group. As another example, R³ and R⁴ may each independently be a substituted or unsubstituted C1 to C7 alkyl group, a substituted or unsubstituted C1 to C5 alkyl group, or a substituted or unsubstituted C1 to C3 alkyl group. Herein, the alkyl group may be chain or branched.

According to one embodiment, in Chemical Formula 2, at least one of R³ or R⁴ may be a C1 to C10 fluoroalkylene group in which one or more hydrogens are substituted with fluorine. In the C1 to C10 fluoroalkylene group in which one or more hydrogens are substituted with fluorine, the number of fluorine substitutions may be, for example, 1 to 21, 2 to 21, 3 to 21, 4 to 21, or 5 to 21. According to a preferred embodiment, the C1 to C10 fluoroalkylene group in which one or more hydrogens are substituted with fluorine may be a C1 to C10 fluoroalkylene group substituted with 3 to 21 fluorines.

According to one embodiment, in Chemical Formula 2, at least one of R³ or R⁴ may be a C1 to C10 perfluoroalkyl group in which all hydrogens are substituted with fluorine. For example, a C1 to C6 perfluoroalkyl group, or a C2 to C5 perfluoroalkyl group. In one example, both (e.g., simultaneously) R³ and R⁴ may each independently be a C1 to C10 perfluoroalkyl group. In some embodiments, if (e.g., when) a fluorinated ketone including a perfluoroalkyl group is utilized, battery flame retardancy may be improved while electrolyte impregnability is increased, and overall performance of the battery may be improved.

In Chemical Formula 2, R³ and R⁴ may be the same as or different from each other. In one example, R³ and R⁴ may each be a C1 to C10 perfluoroalkyl group, but different from each other. In this case, high-speed charging performance at high voltage may be improved while ensuring battery flame retardancy.

In Chemical Formula 2, n is any one of integers from 0 to 5, and may be, for example, any one of integers from 0 to 4, any one of integers from 0 to 3, or any one of integers from 0 to 2, for example, 0 or 1.

As a specific example, the fluorinated ketone may be represented by Chemical Formula 2-1.

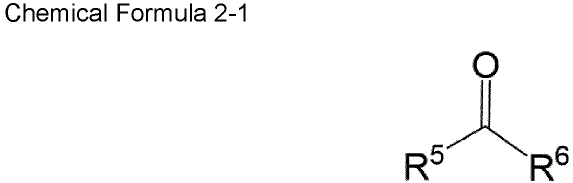

In Chemical Formula 2-1,
R⁵ and R⁶ may each independently be a substituted or unsubstituted C1 to C30 alkyl group, and at least one of R⁵ or R⁶ includes one or more fluorines.

For example, R⁵ and R⁶ may each independently be a C1 to C20 fluoroalkyl group substituted with at least one fluorine. For example, R⁵ and R⁶ may each independently be a C1 to C10 fluoroalkyl group substituted with at least one fluorine. For example, the fluoroalkyl group refers to one in which all or part of the hydrogens of an ethyl group, a propyl group, a butyl group, a pentyl group, a hexyl group, a heptyl group, an octyl group, a nonyl group, or a decyl group are substituted with fluorine. For example, all hydrogens of the ethyl group may be substituted with fluorine. The alkyl group includes all structural isomers, and for example, the propyl group includes an n-propyl group and an iso-propyl group, and the butyl group includes an n-butyl group, an iso-butyl group, a sec-butyl group, and a tert-butyl group.

In some embodiments, the fluoroalkyl group includes those in which all or part of the hydrogens among the above-listed alkyl groups are substituted with fluoroalkyl groups. For example, one of the hydrogens of ethyl may be substituted with a trifluoromethyl group and all other hydrogens (not substituted with the trifluoromethyl group) may be substituted with fluorine. For example, two of the hydrogens of ethylene may be substituted with trifluoromethyl groups, and all of the remaining hydrogens may be substituted with fluorine, but embodiments are not limited thereto. According to one embodiment, at least one of R5 or R6 is a C1 to C10 perfluoroalkyl group in which all hydrogens are substituted with fluorine (F).

In Chemical Formula 2-1, R⁵ and R⁶ may be the same as or different from each other. According to one embodiment, R⁵ and R⁶ of Chemical Formula 2-1 may be different from each other, and thus the compound represented by Chemical Formula 2-1 may have an asymmetric structure. In this case, high-speed charging performance at high voltage can be further improved while securing flame retardancy of the battery.

The fluorinated ketone represented by Chemical Formula 2 may be, for example represented by any one selected from Chemical Formula 2-2 and Chemical Formula 2-3.

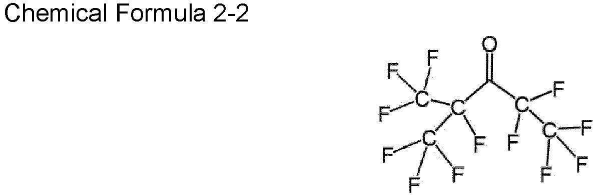

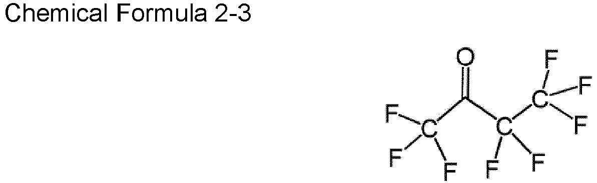

The fluorinated ketone may be included in an amount of 1.0 wt% to 10.0 wt%, 1.5 wt% to 9.0 wt%, 2.0 wt% to 8.5 wt%, 2.5 wt% to 8.0 wt%, 3.0 wt% to 7.5 wt%, 3.5 wt% to 7.0 wt%, 4.0 wt% to 6.5 wt%, based on a total weight of the electrolyte for a rechargeable lithium battery. For example, it may be included in an amount of greater than or equal to 1.5 wt%, for example greater than or equal to 2.0 wt%, for example greater than or equal to 2.5 wt%, for example greater than or equal to 3.0 wt%, for example greater than or equal to 3.5 wt%, or for example greater than or equal to 4.0 wt%, and for example less than or equal to 10.0 wt%, for example less than or equal to 9.0 wt%, for example less than or equal to 8.5 wt%, for example less than or equal to 8.0 wt%, for example less than or equal to 7.5 wt%, or for example less than or equal to 7.0 wt%, but is not limited thereto. By including the fluorinated ketone within the above ranges in the electrolyte according to some embodiments, flame retardant properties may be imparted to the electrolyte and impregnability of the negative electrode of the electrolyte may be improved.

In a preferred embodiment the electrolyte of the present invention may comprise a non-aqueous organic solvent, a lithium salt, and an additive and the additive may comprise as a first compound a compound represented by Chemical Formula 1 and as second compound a fluorinated ketone and an Ag salt. More preferred, the additive may comprise as a first compound a compound represented by Chemical Formula 1 and as second compound a compound represented by Chemical Formula 2-2 or Formula 2-3 and an Ag salt. Especially preferred, the additive may comprise as a first compound a compound represented by Chemical Formula 1-1 and as second compound a compound represented by Chemical Formula 2-2 and an Ag salt. Preferably the Ag salt may be silver nitrate.

In one embodiment, a weight ratio of the fluorinated ketone and the Ag salt may be 1:9 to 6:4, for example 1:1, for example 1:2, for example 1:3, or for example 1:4, but is not limited thereto. The additive may be included in an amount of 1.0 wt% to 30.0 wt%, 2.0 wt% to 25.0 wt%, 3.0 wt% to 20 wt%, 4.0 wt% to 18 wt%, 5.0 wt% to 16 wt%, 6.0 wt% to 15 wt%, based on a total weight of the electrolyte for a rechargeable lithium battery. For example, it may be included in an amount of greater than or equal to 1.0 wt%, for example greater than or equal to 2.0 wt%, for example greater than or equal to 3.0 wt%, for example greater than or equal to 4.0 wt%, or for example greater than or equal to 5.0 wt%, and for example less than or equal to 30.0 wt%, for example less than or equal to 25.0 wt%, for example less than or equal to 20.0 wt%, for example less than or equal to 18.0 wt%, for example less than or equal to 16.0 wt%, or for example less than or equal to 15.0 wt%, but is not limited thereto. By including the additive within the above ranges in the electrolyte according to some embodiments, high-speed charging performance, high-temperature cycle-life characteristics, and safety of a rechargeable lithium battery including the additive may be improved.

The electrolyte for a rechargeable lithium battery according to some embodiments may further include other additives.
According to one embodiment, the electrolyte for the rechargeable lithium battery 100 further comprises other additives, and he other additives may comprise at least one selected from among vinylene carbonate (VC), fluoroethylene carbonate (FEC), difluoroethylene carbonate (DFEC), chloroethylene carbonate (CEC), dichloroethylene carbonate (DCEC), bromoethylene carbonate (BEC), dibromoethylene carbonate (DBEC), nitroethylene carbonate, cyanoethylene carbonate, vinylethylene carbonate (VEC), succinonitrile (SN), adiponitrile (AN), 1,3,6-hexane tricyanide (HTCN), propene sultone (PST), propane sultone (PS), lithium tetrafluoroborate (LiBF₄), lithium difluorophosphate (LiPO₂F₂), 2-fluoro biphenyl (2-FBP), lithium difluorooxalatoborate (LiDFOB), and lithium bisoxalatoborate (LiBOB).

The other additives may be included in an amount of, for example, 0.1 wt% to 30.0 wt%, 1.0 wt% to 20.0 wt%, or 2.0 wt% to 15.0 wt% based on a total weight of the electrolyte for a rechargeable lithium battery. For example, the additives may be included in an amount of greater than or equal to 1.0 wt%, for example greater than or equal to 2.0 wt%, for example greater than or equal to 3.0 wt%, for example greater than or equal to 4.0 wt%, or for example greater than or equal to 5.0 wt%, and for example less than or equal to 30.0 wt%, for example less than or equal to 25.0 wt%, for example less than or equal to 20.0 wt%, for example less than or equal to 18.0 wt%, for example less than or equal to 16.0 wt%, or for example less than or equal to 15.0 wt%, but are not limited thereto. By including other additives within the above range in the electrolyte according to some embodiments, high-speed charging performance, high-temperature cycle-life characteristics, and stability of a rechargeable lithium battery including the additives may be improved.

### Non-aqueous Organic Solvent

The non-aqueous organic solvent serves as a medium for transmitting ions taking part in the electrochemical reaction of a battery.

The non-aqueous organic solvent may include a carbonate-based, ester-based, ether-based, ketone-based, alcohol-based, or aprotic solvent.

The carbonate-based solvent may be dimethyl carbonate (DMC), diethyl carbonate (DEC), dipropyl carbonate (DPC), methylpropyl carbonate (MPC), ethylpropyl carbonate (EPC), methylethyl carbonate (MEC), ethylene carbonate (EC), propylene carbonate (PC), butylene carbonate (BC), and/or the like. The ester-based solvent may include methyl acetate, ethyl acetate, n-propyl acetate, t-butyl acetate, methylpropionate, ethyl propionate, propyl propionate, decanolide, mevalonolactone, caprolactone, and/or the like. The ether-based solvent may include dibutyl ether, tetraglyme, diglyme, dimethoxyethane, 2-methyltetrahydrofuran, tetrahydrofuran, and/or the like. The ketone-based solvent may include cyclohexanone and/or the like. The alcohol-based solvent may include ethyl alcohol, isopropyl alcohol, and/or the like, and examples of the aprotic solvent include nitriles such as R-CN (wherein R is a C2 to C20 linear, branched, or a cyclic hydrocarbon group, a double bond, an aromatic ring, or an ether bond), amides such as dimethylformamide, dioxolanes such as 1,3-dioxolane, sulfolanes, and/or the like.

The non-aqueous organic solvent may be utilized alone or in combination with one or more of them, and if (e.g., when) utilized in combination with one or more, a mixing ratio may be appropriately adjusted according to the desired or suitable battery performance, which is well understood by those skilled in the art.

In some embodiments, in the case of the carbonate-based solvent, a mixture of cyclic carbonate and chain carbonate may be utilized. In this case, if (e.g., when) the cyclic carbonate and the chain carbonate are mixed in a volume ratio of 1:1 to 1:9, the performance of the electrolyte may be improved.

The non-aqueous organic solvent may further include an aromatic hydrocarbon-based organic solvent. The carbonate-based solvent and aromatic hydrocarbon-based solvent may be mixed together in a volume ratio of 1:1 to 30:1.

The aromatic hydrocarbon-based solvent may be an aromatic hydrocarbon-based compound represented by Chemical Formula 3.

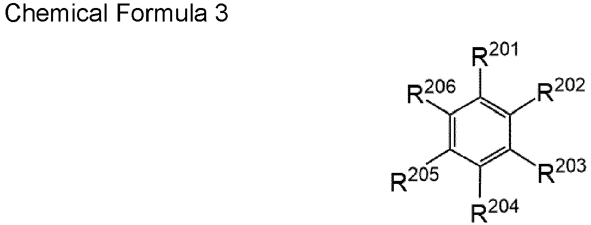

In Chemical Formula 3, R²⁰¹ to R²⁰⁶ may each independently be the same or different and are hydrogen, a halogen, a C1 to C10 alkyl group, a haloalkyl group, or a combination thereof.

Examples of the aromatic hydrocarbon-based organic solvent may be benzene, fluorobenzene, 1,2-difluorobenzene, 1,3-difluorobenzene, 1,4-difluorobenzene, 1,2,3-trifluorobenzene, 1,2,4-trifluorobenzene, chlorobenzene, 1,2-dichlorobenzene, 1,3-dichlorobenzene, 1,4-dichlorobenzene, 1,2,3-trichlorobenzene, 1,2,4-trichlorobenzene, iodobenzene, 1,2-diiodobenzene, 1,3-diiodobenzene, 1,4-diiodobenzene, 1,2,3-triiodobenzene, 1,2,4-triiodobenzene, toluene, fluorotoluene, 2,3-difluorotoluene, 2,4-difluorotoluene, 2,5-difluorotoluene, 2,3,4-trifluorotoluene, 2,3,5-trifluorotoluene, chlorotoluene, 2,3-dichlorotoluene, 2,4-dichlorotoluene, 2,5-dichlorotoluene, 2,3,4-trichlorotoluene, 2,3,5-trichlorotoluene, iodotoluene, 2,3-diiodotoluene, 2,4-diiodotoluene, 2,5-diiodotoluene, 2,3,4-triiodotoluene, 2,3,5-triiodotoluene, xylene, and/or one or more combinations thereof.

The electrolyte may further include vinylene carbonate, vinyl ethylene carbonate, or an ethylene carbonate-based compound represented by Chemical Formula 4 in order to improve cycle-life of a battery.

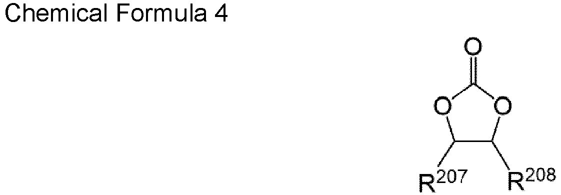

In Chemical Formula 4, R²⁰⁷ and R²⁰⁸ may each independently be the same or different, and selected from among hydrogen, a halogen, a cyano group (CN), a nitro group (NO₂), and a fluorinated C1 to C5 alkyl group.

Examples of the ethylene carbonate-based compound may be difluoro ethylene carbonate, chloroethylene carbonate, dichloroethylene carbonate, bromoethylene carbonate, dibromoethylene carbonate, nitroethylene carbonate, cyanoethylene carbonate, and/or fluoroethylene carbonate. The amount of the additive for improving cycle-life may be utilized within an appropriate or suitable range.

### Lithium Salt

The lithium salt dissolved in the non-organic solvent supplies lithium ions in a battery, enables a basic operation of a rechargeable lithium battery, and improves transportation of the lithium ions between positive and negative electrodes.

Examples of the lithium salt include at least one selected from among LiPF₆, LiBF₄, LiSbF₆, LiAsF₆, LiN(SO₂C₂F₅)₂, Li(CF₃SO₂)₂N, LiN(SO₃C₂F₅)₂, Li(FSO₂)₂N (lithium bis(fluorosulfonyl)imide, LiFSI), LiC₄F₉SO₃, LiClO₄, LiAlO₂, LiAlCl₄, LiPO₂F₂, LiN(CₓF₂ₓ₊₁SO₂)(C_{y}F_{2y+1}SO₂), wherein, x and y are natural numbers, for example, an integer in a range of 1 to 20, lithium difluoro(bisoxalato) phosphate, LiCl, Lil, LiB(C₂O₄)₂ (lithium bis(oxalato) borate, LiBOB), and lithium difluoro (oxalato)borate (LiDFOB).

The lithium salt may be utilized in a concentration in a range of 0.1 M to 2.0 M. If (e.g., when) the lithium salt is included at the above concentration range, an electrolyte may have excellent or suitable performance and lithium ion mobility due to optimal or suitable electrolyte conductivity (e.g., ion conductivity) and viscosity.

### Rechargeable Lithium Battery

The additive and the electrolyte may be applied to a rechargeable lithium battery 100.

The rechargeable lithium battery 100 according to some embodiments includes a positive electrode 114 including a positive electrode active material; a negative electrode 112 including a negative electrode active material; a separator 113 between the positive electrode 114 and the negative electrode 112; and the aforementioned electrolyte.

Rechargeable lithium batteries may be classified as lithium ion batteries, lithium ion polymer batteries, and/or lithium polymer batteries according to the presence of a separator and the type or kind of electrolyte utilized therein. The rechargeable lithium batteries may have a variety of shapes and sizes, and examples of the shapes include cylindrical, prismatic, coin, and/or pouch-type or kind batteries, and examples of the sizes may be thin film batteries and/or may be rather bulky in size. Structures and manufacturing methods for lithium ion batteries pertaining to this disclosure are well suitable in the art.

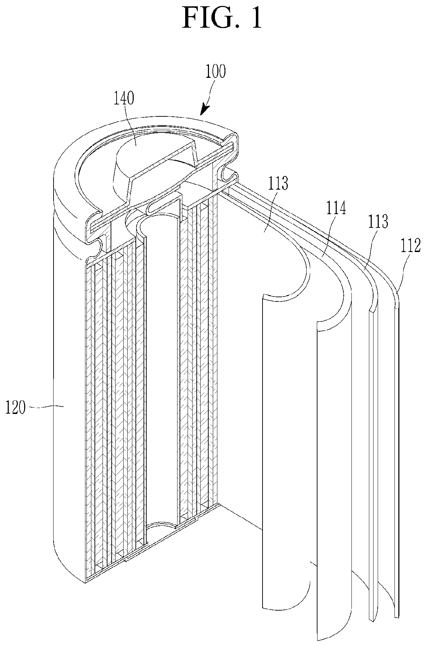

Herein, a cylindrical rechargeable lithium battery will be exemplarily described as an example of the rechargeable lithium battery 100. FIG. 1 schematically shows the structure of a rechargeable lithium battery 100 according to some embodiments. Referring to FIG. 1, a rechargeable lithium battery 100 according to some embodiments includes a battery cell including a positive electrode 114, a negative electrode 112 facing the positive electrode 114, a separator 113 between the positive electrode 114 and the negative electrode 112, and an electrolyte impregnating the positive electrode 114, the negative electrode 112, and the separator 113, a battery case 120 housing the battery cell, and a sealing member 140 sealing the battery case 120.

### Positive Electrode

The positive electrode 114 includes a positive electrode current collector and a positive electrode active material layer on the positive electrode current collector, and the positive electrode active material layer includes a positive electrode active material.

The positive electrode active material may include a compound (lithiated intercalation compound) capable of intercalating and deintercallating lithium, and for example, a compound represented by one of the following chemical formulas.

LiₐA_{1-b}X_{b}D₂ (0.90 ≤ a ≤ 1.8, 0 ≤ b ≤ 0.5);

LiₐA_{1-b}X_{b}O_{2-c}D_{c} (0.90 ≤ a ≤ 1.8, 0 ≤ b ≤ 0.5, 0 ≤ c ≤ 0.05);

LiₐE_{1-b}X_{b}O_{2-c}D_{c} (0.90 ≤ a ≤ 1.8, 0 ≤ b ≤ 0.5, 0 ≤ c ≤ 0.05);

LiₐE_{2-b}X_{b}O_{4-c}D_{c} (0.90 ≤ a ≤ 1.8, 0 ≤ b ≤ 0.5, 0 ≤ c ≤ 0.05);

LiₐNi_{1-b-c}Co_{b}X_{c}D_{α} (0.90 ≤ a ≤1.8, 0 ≤ b ≤ 0.5, 0 ≤ c ≤ 0.5, 0 <α ≤ 2);

LiₐNi_{1-b-c}Co_{b}X_{c}O_{2-α}T_{α} (0.90 ≤ a ≤ 1.8, 0 ≤ b ≤ 0.5, 0 ≤ c ≤ 0.05, 0 <α <2);

LiₐNi_{1-b-c}Co_{b}X_{c}O_{2-α}T₂ (0.90 ≤ a ≤ 1.8, 0 ≤ b ≤ 0.5, 0 ≤ c ≤ 0.05, 0 <α <2);

LiₐNi_{1-b-c}Mn_{b}X_{c}D_{α} (0.90 ≤ a ≤ 1.8, 0 ≤ b ≤ 0.5, 0 ≤ c ≤ 0.05, 0 <α ≤ 2);

LiₐNi_{1-b-c}Mn_{b}X_{c}O_{2-α}T_{α} (0.90 ≤ a ≤ 1.8, 0 ≤ b ≤ 0.5, 0 ≤ c ≤ 0.05, 0 <α < 2);

LiₐNi_{1-b-c}Mn_{b}X_{c}O_{2-α}T₂ (0.90 ≤ a ≤ 1.8, 0 ≤ b ≤ 0.5, 0 ≤ c ≤ 0.05, 0 <α < 2);

LiₐNi_{b}E_{c}G_{d}O₂ (0.90 ≤ a ≤ 1.8, 0 ≤ b ≤ 0.9, 0 ≤ c ≤ 0.5, 0.001 ≤ d ≤ 0.1);

LiₐNi_{b}Co_{c}Mn_{d}GₑO₂ (0.90 ≤ a ≤ 1.8, 0 ≤ b ≤ 0.9, 0 ≤ c ≤ 0.5, 0 ≤ d ≤0.5, 0.001 ≤ e ≤ 0.1);

LiₐNiG_{b}O₂ (0.90 ≤ a ≤ 1.8, 0.001 ≤ b ≤ 0.1);

LiₐCoG_{b}O₂ (0.90 ≤ a ≤ 1.8, 0.001 ≤ b ≤ 0.1);

LiₐMn_{1-b}G_{b}O₂ (0.90 ≤ a ≤ 1.8, 0.001 ≤ b ≤ 0.1);

LiₐMn₂G_{b}O₄ (0.90 ≤ a ≤ 1.8, 0.001 ≤ b ≤ 0.1);

LiₐMn_{1-g}G_{g}PO₄ (0.90 ≤ a ≤ 1.8, 0 ≤ g ≤ 0.5);

QO₂; QS₂; LiQS₂;

V₂O₅; LiV₂O₅;

LiZO₂;

LiNiVO₄;

Li_{(3-f)}J₂(PO₄)₃ (0 ≤ f ≤ 2);

Li_{(3-f)}Fe₂(PO₄)₃ (0 ≤ f ≤ 2);

and/or

LiₐFePO₄ (0.90 ≤ a ≤ 1.8).

In the above chemical formulas, A is selected from among Ni, Co, Mn, and a combination thereof; X is selected from among Al, Ni, Co, Mn, Cr, Fe, Mg, Sr, V, a rare earth element, and a combination thereof; D is selected from among O, F, S, P, and a combination thereof; E is selected from among Co, Mn, and a combination thereof; T is selected from among F, S, P, and a combination thereof; G is selected from among Al, Cr, Mn, Fe, Mg, La, Ce, Sr, V, and a combination thereof; Q is selected from among Ti, Mo, Mn, and a combination thereof; Z is selected from among Cr, V, Fe, Sc, Y, and a combination thereof; and J is selected from among V, Cr, Mn, Co, Ni, Cu, and a combination thereof.

The positive electrode active material may be, for example, lithium cobalt oxide (LCO), lithium nickel oxide (LNO), lithium nickel cobalt oxide (NC), lithium nickel cobalt aluminum oxide (NCA), lithium nickel cobalt manganese oxide (NCM), lithium nickel manganese oxide (NM), lithium manganese oxide (LMO), or lithium iron phosphate oxide (LFP).

The positive electrode active material may be one having a coating layer on the surface of the composite oxide, or a mixture of the composite oxide and the composite oxide having a coating layer. The coating layer may include a coating element compound selected from among an oxide of a coating element, a hydroxide of a coating element, an oxyhydroxide of a coating element, an oxycarbonate of a coating element, and/or a hydroxycarbonate of a coating element. The compound for the coating layer may be either amorphous or crystalline. The coating element included in the coating layer may be Mg, Al, Co, K, Na, Ca, Si, Ti, V, Sn, Ge, Ga, B, As, Zr, or a mixture thereof. The coating process may include any processes as long as it does not cause (or does not substantially cause) any side effects on the properties of the positive electrode active material (e.g., spray coating, dipping), which is well suitable to persons having ordinary skill in this art, and thus a detailed description thereof is omitted.

The positive electrode active material may be, for example, at least one of lithium composite oxides represented by Chemical Formula 5.

Chemical Formula 5 LiₓM¹_{y}M²_{z}M³_{1-y-z}O₂

In Chemical Formula 5,
0.5≤x≤1.8, 0<y≤1, 0≤z≤1, 0≤y+z≤1, and M¹, M², and M³ may each independently be selected from among metal(s) of Ni, Co, Mn, Al, Sr, Mg, and/or La, and combinations thereof.

In some embodiments, the positive electrode active material may be at least one selected from among LiCoO₂, LiNiO₂, LiMnO₂, LiMn₂O₄, LiNiₐMn_{b}Co_{c}O₂ (a+b+c=1), LiNiₐMn_{b}Co_{c}Al_{d}O₂ (a+b+c+d=1), and/or LiNiₑCo_{f}Al_{g}O₂ (e+f+g=1).

For example, the positive electrode active material selected from among LiNiₐMn_{b}Co_{c}O₂ (a+b+c=1), LiNiₐMn_{b}Co_{c}Al_{d}O₂ (a+b+c+d=1), and LiNiₑCo_{f}Al_{g}O₂ (e+f+g=1) may be a high-Ni positive electrode active material.

For example, in the case of LiNiₐMn_{b}Co_{c}O₂ (a+b+c=1) and LiNiₐMn_{b}Co_{c}Al_{d}O₂ (a+b+c+d=1), a nickel content (e.g., amount) may be greater than or equal to 60% (a ≥ 0.6), and more specifically, greater than or equal to 80% (a ≥ 0.8).

For example, in the case of LiNiₑCo_{f}Al_{g}O₂ (e+f+g=1), a nickel content (e.g., amount) may be greater than or equal to 60% (a ≥ 0.6), and more specifically, greater than or equal to 80% (a ≥ 0.8).

An amount of the positive electrode active material may be 90 wt% to 98 wt% based on a total weight of the positive electrode active material layer.

The positive electrode active material layer may optionally include a conductive material and a binder. In this case, each content (e.g., amount) of the conductive material and the binder may be 1.0 wt% to 5.0 wt%, based on a total weight of the positive electrode active material layer. It may be 1 wt% to 5 wt% based on the total weight of the positive electrode active material layer.

The conductive material is utilized to impart conductivity to the negative electrode, and any electrically conductive material may be utilized as a conductive material unless it causes a chemical change in a battery. Examples of the conductive material may include a carbon-based material such as natural graphite, artificial graphite, carbon black, acetylene black, ketjen black, carbon fiber, carbon nanofiber, carbon nanotube, and/or the like; a metal-based material of a metal powder or a metal fiber including copper, nickel, aluminum, silver, and/or the like; a conductive polymer such as a polyphenylene derivative; and/or one or more mixtures thereof.

The binder improves binding properties of positive electrode active material particles with one another and with a current collector. Examples thereof may be polyvinyl alcohol, carboxylmethyl cellulose, hydroxypropyl cellulose, diacetyl cellulose, polyvinylchloride, carboxylated polyvinylchloride, polyvinylfluoride, an ethylene oxide-containing polymer, polyvinylpyrrolidone, polyurethane, polytetrafluoroethylene, polyvinylidene fluoride, polyethylene, polypropylene, a styrene-butadiene rubber, an acrylated styrene-butadiene rubber, an epoxy resin, nylon, and/or the like, but are not limited thereto.

The positive electrode current collector may be aluminum foil or SUS foil, but is not limited thereto.

### Negative Electrode

The negative electrode 112 includes a negative electrode current collector and a negative electrode active material layer including the negative electrode active material on the negative electrode current collector.

The negative electrode active material includes a material capable of reversibly intercalating/deintercalating lithium ions, lithium metal, a lithium metal alloy, a material capable of doping and undoping lithium, or a transition metal oxide.

The material that reversibly intercalates/deintercalates lithium ions includes carbon materials. The carbon material may be any generally-utilized carbon-based negative electrode active material in a rechargeable lithium battery. Examples of the carbon material include crystalline carbon, amorphous carbon, and a combination thereof. The crystalline carbon may be non-shaped, or sheet, flake, spherical, and/or fiber shaped natural graphite and/or artificial graphite. The amorphous carbon may be a soft carbon, a hard carbon, a mesophase pitch carbonized product, fired coke, and/or the like.

The lithium metal alloy may include lithium and a metal selected from among Na, K, Rb, Cs, Fr, Be, Mg, Ca, Sr, Si, Sb, Pb, In, Zn, Ba, Ra, Ge, Al, and Sn.

The material capable of doping and dedoping lithium may include Si, SiOₓ (0 < x < 2), a Si-Q alloy (wherein Q is selected from among an alkali metal, an alkaline-earth metal, a Group 13 element, a Group 14 element, a Group 15 element, a Group 16 element, a transition metal, a rare earth element, and a combination thereof, and not Si), Sn, SnO₂, a Sn-R alloy (wherein R is an alkali metal, an alkaline-earth metal, a Group 13 element, a Group 14 element, a Group 15 element, a Group 16 element, a transition element, a rare earth element, or a combination thereof, and not Sn), and/or the like. At least one of them may be mixed with SiO₂.

The elements Q and R may be selected from among Mg, Ca, Sr, Ba, Ra, Sc, Y, Ti, Zr, Hf, Rf, V, Nb, Ta, Db, Cr, Mo, W, Sg, Tc, Re, Bh, Fe, Pb, Ru, Os, Hs, Rh, Ir, Pd, Pt, Cu, Ag, Au, Zn, Cd, B, Al, Ga, Sn, In, Tl, Ge, P, As, Sb, Bi, S, Se, Te, Po, and combinations thereof.

The transition metal oxide may be a vanadium oxide, a lithium vanadium oxide, and/or the like.

In some embodiments, the negative electrode active material may be a Si-C composite including a Si-based active material and a carbon-based active material.

An average particle diameter of the Si-based active material in the Si-C composite may be 50 nm to 200 nm. If (e.g., when) the average particle diameter of the Si-based active material is within the above range, volume expansion occurring during charging and discharging may be suppressed or reduced, and a break in a conductive path due to particle crushing during charging and discharging may be prevented or reduced. The Si-based active material may be included in an amount of 1 to 60 wt%, for example, 3 to 60 wt% based on the total weight of the Si-C composite.

In some embodiments, the negative electrode active material may further include crystalline carbon together with the aforementioned Si-C composite.

If (e.g., when) the negative electrode active material includes a Si-C composite and crystalline carbon together, the Si-C composite and crystalline carbon may be included in the form of a mixture, and in this case, the Si-C composite and crystalline carbon may be included in a weight ratio of 1:99 to 50:50. More specifically, the Si-C composite and crystalline carbon may be included in a weight ratio of 5 : 95 to 20 : 80.

The crystalline carbon may include for example graphite, and more specifically natural graphite, artificial graphite, or a mixture thereof. The crystalline carbon may have an average particle diameter of 5 µm to 30 µm.

As utilized herein, the average particle diameter may be a particle size (D50) at a volume ratio of 50% in a cumulative size-distribution curve.

The Si-C composite may further include a shell around (e.g., surrounding) a surface of the Si-C composite, and the shell may include amorphous carbon. The amorphous carbon may include soft carbon, hard carbon, a mesophase pitch carbonized product, calcined coke, or a mixture thereof.

The amorphous carbon may be included in an amount of 1 to 50 parts by weight, for example, 5 to 50 parts by weight, or 10 to 50 parts by weight, based on 100 parts by weight of the carbon-based active material.

In the negative electrode active material layer, the negative electrode active material may be included in an amount of 95 wt% to 99 wt% based on a total weight of the negative electrode active material layer.

In some embodiments, the negative electrode active material layer may include a binder, and optionally a conductive material. In the negative electrode active material layer, the amount of the binder may be 1 wt% to 5 wt% based on a total weight of the negative electrode active material layer. If (e.g., when) the negative electrode active material layer further includes the conductive material, it may include 90 wt% to 98 wt% of the negative electrode active material, 1 wt% to 5 wt% of the binder, and 1 wt% 5 wt% of the conductive material.

The binder improves binding properties of negative electrode active material particles with one another and with a current collector. The binder may be a non-water-soluble binder, a water-soluble binder, or a combination thereof.

The non-water-soluble binder may be polyvinylchloride, carboxylated polyvinylchloride, polyvinylfluoride, polyurethane, polytetrafluoroethylene, polyvinylidene fluoride, polyethylene, polypropylene, polyamideimide, polyimide, or a combination thereof.

The water-soluble binder may be a rubber-based binder or a polymer resin binder. The rubber-based binder may be selected from among a styrene-butadiene rubber(SBR), an acrylated styrene-butadiene rubber, an acrylonitrile-butadiene rubber, an acrylic rubber, a butyl rubber, a fluorine rubber, and a combination thereof. The polymer resin binder may be selected from among polytetrafluoroethylene, ethylene propylene copolymer, polyethylene oxide, polyvinylpyrrolidone, polyepichlorohydrine, polyphosphazene, polyacrylonitrile, polystyrene, an ethylene propylenediene copolymer, polyvinylpyridine, chlorosulfonated polyethylene, latex, a polyester resin, an acrylic resin, a phenolic resin, an epoxy resin, polyvinyl alcohol, and a combination thereof.

If (e.g., when) the water-soluble binder is utilized as the negative electrode binder, a cellulose-based compound may be further utilized to provide viscosity as a thickener. The cellulose-based compound includes one or more of carboxylmethyl cellulose, hydroxypropylmethyl cellulose, methyl cellulose, or alkali metal salts thereof. The alkali metal may be Na, K, or Li. Such a thickener may be included in an amount of 0.1 parts by weight to 3 parts by weight based on 100 parts by weight of the negative electrode active material.

The conductive material is included to provide electrode conductivity and any electrically conductive material may be utilized as a conductive material unless it causes a chemical change. Examples thereof may be a carbon-based material such as natural graphite, artificial graphite, carbon black, acetylene black, ketjen black, carbon fiber, carbon nanofiber, carbon nanotube, and/or the like; a metal-based material such as a metal powder or a metal fiber of copper, nickel, aluminum, silver, and/or the like; a conductive polymer such as a polyphenylene derivative and/or the like, and/or one or more mixtures thereof.

The negative electrode current collector may be selected from among a copper foil, a nickel foil, a stainless steel foil, a titanium foil, a nickel foam, a copper foam, a polymer substrate coated with a conductive metal, and a combination thereof.

### Separator

The separator 113 separates the positive electrode 114 and the negative electrode 112 and provides a passage for lithium ions to move, and any one commonly utilized in a lithium ion battery may be utilized. The separator may have low resistance to ion movement of the electrolyte and excellent or suitable ability to absorb the electrolyte. For example, the separator may include a glass fiber, polyester, polyethylene, polypropylene, polytetrafluoroethylene, or a combination thereof and may have a form of a non-woven fabric or a woven fabric. For example, in a lithium ion battery, a polyolefin-based polymer separator such as polyethylene and polypropylene may be mainly utilized. In order to ensure the heat resistance or mechanical strength, a coated separator including a ceramic component or a polymer material may be utilized. Optionally, it may have a mono-layered or multi-layered structure.

Examples of a suitable separator material include polyethylene, polypropylene, polyvinylidene fluoride, and multi-layers thereof such as a polyethylene/polypropylene double-layered separator, a polyethylene/polypropylene/polyethylene triple-layered separator, and a polypropylene/polyethylene/polypropylene triple-layered separator.

The separator may have a form including a functional layer on a porous substrate. The functional layer may be, for example, at least one of a heat-resistant layer or an adhesive layer from the viewpoint of enabling additional function. For example, the heat-resistant layer may include a heat-resistant resin and optionally a filler. In some embodiments, the adhesive layer may include an adhesive resin and optionally a filler. The filler may be an organic filler or an inorganic filler.

Terms such as "substantially," and "approximately" are used as relative terms and not as terms of degree, and are intended to account for the inherent deviations in measured or calculated values that would be recognized by those of ordinary skill in the art. They may be inclusive of the stated value and an acceptable range of deviation as determined by one of ordinary skill in the art, considering the limitations and error associated with measurement of that quantity.

Numerical ranges disclosed herein include and are intended to disclose all subsumed sub-ranges of the same numerical precision. For example, a range of "1.0 to 10.0" includes all subranges having a minimum value equal to or greater than 1.0 and a maximum value equal to or less than 10.0, such as, for example, 2.4 to 7.6. Applicant therefore reserves the right to amend this specification, including the claims, to expressly recite any sub-range subsumed within the ranges expressly recited herein. In the present disclosure, particles are spherical, "diameter" indicates a particle diameter or an average particle diameter, and when the particles are non-spherical, the "diameter" indicates a major axis length or an average major axis length.

Hereinafter, the present disclosure is illustrated in more detail with reference to examples. However, these examples are example, and the present disclosure is not limited thereto.

### Examples

### Preparation of Electrolyte

### Comparative Preparation Example 1

LiPF₆ was added to a mixed solvent of ethylene carbonate (EC), propylene carbonate (PC), and propylpropionate (PP) that were mixed in a volume ratio of 10:15:75, preparing a 1.3 M LiPF₆ mixed solution. 7 parts by weight of fluoroethylene carbonate (FEC), 1 part by weight of vinylethylene carbonate (VEC), and 0.2 parts by weight of LiBF₄ based on 100 parts by weight of the mixed solution electrolyte were added thereto, preparing an electrolyte.

### Comparative Preparation Example 2

An electrolyte was prepared in substantially the same manner as in Comparative Preparation Example 1, except that 0.5 parts by weight of the compound represented by Chemical Formula 1-1 was added based on 100 parts by weight of the mixed solution.

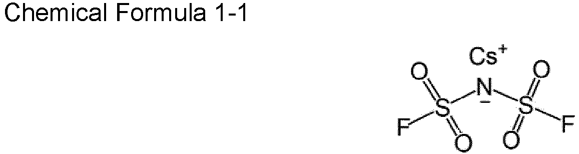

### Comparative Preparation Example 3

An electrolyte was prepared in substantially the same manner as in Comparative Preparation Example 1 except that 1 part by weight of silver nitrate based on 100 parts by weight of the mixed solution was added.

### Comparative Preparation Example 4

An electrolyte was prepared in substantially the same manner as in Comparative Preparation Example 1 except that 0.5 parts by weight of a compound represented by Chemical Formula 2-2 as fluorinated ketone based on 100 parts by weight of the mixed solution was added.

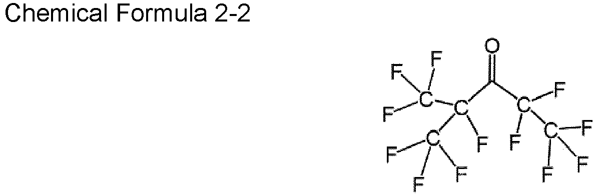

### Preparation Example 1

An electrolyte was prepared in substantially the same manner as in Comparative Preparation Example 1 except that 0.5 parts by weight of a compound represented by Chemical Formula 1-1 and 1 part by weight of silver nitrate based on 100 parts by weight of the mixed solution were added.

### Preparation Example 2

An electrolyte was prepared in substantially the same manner as in Preparation Example 1 except that 0.5 parts by weight of the compound represented by Chemical Formula 2-2 based on 100 parts by weight of the mixed solution was added.

### Manufacture of Rechargeable Lithium Battery Cells

### Example 1

### (Manufacture of Negative Electrode)

98 wt% of artificial graphite (BSG-L, Tianjin BTR New Energy Technology Co., Ltd.), 1.0 wt% of a styrene-butadiene rubber (SBR) binder (ZEON), and 1.0 wt% of carboxylmethyl cellulose (CMC, NIPPON A&L) were mixed and added to distilled water and then, stirred for 60 minutes by utilizing a mechanical stirrer, preparing negative electrode active material slurry. The slurry was coated to be about 60 micrometer (µm) thick on a 10 µm-thick copper current collector by utilizing a doctor blade, dried in a 100 °C forced convection drying oven for 0.5 hour, dried again under vacuum at 120 °C for 4 hours, and roll-pressed, manufacturing a negative electrode plate. Herein, negative electrode had density in active material of 1.6 gram per cubic centimeter (g/cc).

### (Manufacture of Positive Electrode)

96 wt% of LiCoO₂, 2 wt% of carbon black (Ketjenblack, ECP) as a conductive material, and 2 wt% of polyvinylidene fluoride (PVdF, S6020, Solvay) as a binder were added to an N-methyl-2-pyrrolidone solvent and then, stirred for 30 minutes by utilizing a mechanical stirrer, preparing positive electrode active material slurry. The slurry was coated to be about 60 µm thick on a 20 µm-thick aluminum current collector by utilizing a doctor blade, dried in a 100 °C forced convection drying oven for 0.5 hour, dried again under vacuum at 120 °C for 4 hours, and roll-pressed, manufacturing a positive electrode plate.

### (Manufacture of Battery Cells)

The manufactured positive and negative electrodes are assembled with a 25 µm-thick polyethylene separator to obtain an electrode assembly, and the electrolyte of Preparation Example 1 was injected thereinto, manufacturing a rechargeable lithium battery cell. The rechargeable lithium battery cell had an operation voltage of 4.5 V and a charge rate of 3.0 C.

### Example 2

A rechargeable lithium battery cell was manufactured in substantially the same manner as in Example 1 except that the electrolyte of Preparation Example 2 was utilized.

### Comparative Example 1

A rechargeable lithium battery cell was manufactured in substantially the same manner as in Example 1 except that the electrolyte of Comparative Preparation Example 1 was utilized, the negative electrode density in active material was 1.5 g/cc, and the battery charge rate was 1.3 C.

### Comparative Example 2

A rechargeable lithium battery cell was manufactured in substantially the same manner as in Example 1 except that the electrolyte of Comparative Preparation Example 1 was utilized, and the battery charge rate was 1.3 C.

### Comparative Example 3

A rechargeable lithium battery cell was manufactured in substantially the same manner as in Example 1 except that the electrolyte of Comparative Preparation Example 1 was utilized.

### Comparative Example 4

A rechargeable lithium battery cell was manufactured in substantially the same manner as in Example 1 except that the electrolyte of Comparative Preparation Example 2 was utilized.

### Comparative Example 5

A rechargeable lithium battery cell was manufactured in substantially the same manner as in Example 1 except that the electrolyte of Comparative Preparation Example 3 was utilized.

### Comparative Example 6

A rechargeable lithium battery cell was manufactured in substantially the same manner as in Example 1 except that the electrolyte of Comparative Preparation Example 4 was utilized.

The additive composition(s) (e.g., each additive composition) of the rechargeable lithium battery cells of Examples 1 to 2 and Comparative Examples 1 to 6 are provided in Table 1.

**Table 1**

| | Negative electrode active material density (g/cc) | Charge rate | Composition of electrolyte | | |
|---|---|---|---|---|---|
| | | | CsFSI (parts by weight) | AgNO₃ (parts by weight) | PMP (parts by weight) |
| Comp. Ex.1 | 1.5 | 1.3 C | - | - | - |
| Comp. Ex.2 | 1.6 | 1.3 C | - | - | - |
| Comp. Ex.3 | | 3.0 C | - | - | - |
| Comp. Ex.4 | | | 0.5 | - | - |
| Comp. Ex.5 | | | - | 1 | - |
| Comp. Ex.6 | | | - | - | 0.5 |
| Ex.1 | | | 0.5 | 1 | - |
| Ex. 2 | | | 0.5 | 1 | 0.5 |

### Evaluation 1: Evaluation of Dendrite Generation

A LiCoO₂ positive electrode, a graphite negative electrode, and a separator were utilized and manufactured into a 2032 coin cell, and the cell was charged to 4.6 V (CC/CV 0.2 C 4.6 V, 0.05 C cutoff) and then, dissembled to examine whether or not lithium dendrites were generated at each of the negative electrodes.

The results are shown in Table 2.

**Table 2**

| | Generation of lithium dendrite |
|---|---|
| Comp. Ex.1 | X |
| Comp. Ex.2 | X |
| Comp. Ex.3 | O |
| Comp. Ex.4 | O |
| Comp. Ex.5 | O |
| Comp. Ex.6 | O |
| Ex.1 | X |
| Ex. 2 | X |

Referring to Table 2, the rechargeable lithium battery cells according to Examples 1 and 2 were suppressed or protected from generation of lithium dendrites, (i.e., "X" in Table 2) but Comparative Examples 3 to 6 having the same negative electrode active material density and charge rate as those of the examples exhibited generation of lithium dendrites (i.e., "O" in Table 2). Accordingly, a formation of lithium dendrites in each of the rechargeable lithium battery cells of the examples was suppressed or reduced by utilizing the electrolyte according to some embodiments and the rechargeable lithium battery cells also each exhibited increased battery safety.

### Evaluation 2: Evaluation of High-temperature Cycle-life Characteristics

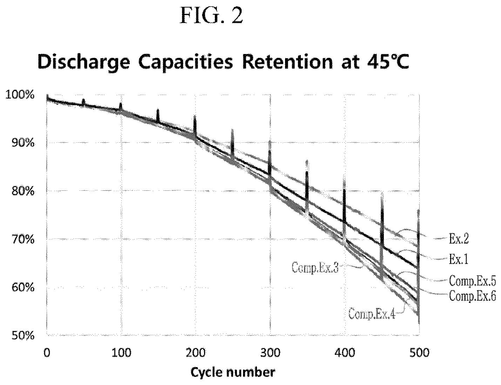

The rechargeable lithium battery cells according to Examples 1 to 2 and Comparative Examples 3 to 6 were each constant current-charged to 4.5 V at 3.0 C and subsequently, constant current-discharged to 3.0 V at a high temperature (45 °C), which were 500 cycles repeated and then, measured with respect to discharge capacity to compare discharge capacity retention and thus evaluate high-temperature cycle-life characteristics. Table 3 shows the measured discharge capacity, and FIG. 2 is a graph showing changes in discharge capacity retentions according to charge and discharge cycles.

**Table 3**

| | 4.5V high temperature cycle-life @ 500^{th} cycle (%) |
|---|---|
| Comparative Example3 | 55 |
| Comparative Example4 | 57 |
| Comparative Example5 | 59 |
| Comparative Example6 | 57 |
| Example1 | 64 |
| Example 2 | 69 |

Referring to Table 3 and FIG. 2, the rechargeable lithium battery cells of Examples 1 and 2 exhibited excellent or suitable discharge capacity retention, compared with the rechargeable lithium battery cells of Comparative Examples 3 to 6. Some embodiments of the present disclosure concurrently (e.g., simultaneously) utilized CsFSI and an Ag salt as electrolyte additives and exhibited improved high-speed charging performance under relatively high density and high voltage conditions.

While this present disclosure has been described in connection with what is presently considered to be practical example embodiments, it is to be understood that the present disclosure is not limited to the disclosed embodiments.

### Description of Symbols

100: rechargeable lithium battery
112: negative electrode
113: separator
114: positive electrode
120: battery case
140: sealing member

## Claims

1. An electrolyte for a rechargeable lithium battery (100), the electrolyte comprising
a non-aqueous organic solvent, a lithium salt, and an additive, wherein the additive comprises:
a first compound selected from among a compound represented by Chemical Formula 1, CsPF₆, and a combination thereof; and
Ag salt, 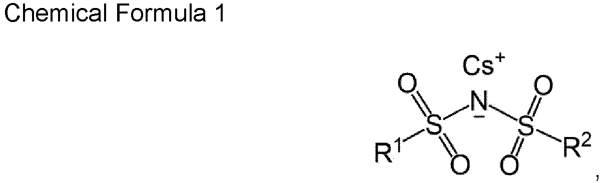
wherein, in Chemical Formula 1,
R¹ and R² are each independently a fluoro group or a substituted C1 to C10 fluoroalkyl group containing at least one fluorine (F).

2. The electrolyte as claimed in claim 1, wherein the first compound comprises the compound represented by Chemical Formula 1, and
wherein R¹ and R² in Chemical Formula 1 are each independently a fluoro group or a C1 to C4 fluoroalkyl group substituted with at least three fluoro groups.

3. The electrolyte as claimed in claim 1 or 2, wherein the first compound comprises the compound represented by Chemical Formula 1, and
wherein Chemical Formula 1 is represented by any one of Chemical Formula 1-1 or Chemical Formula 1-2:

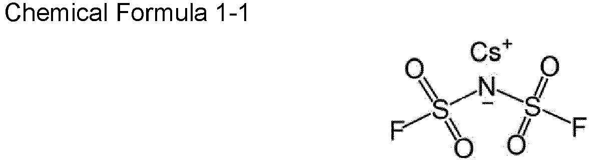

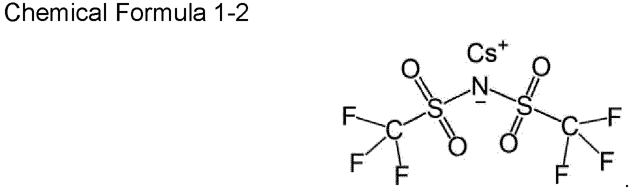

4. The electrolyte as claimed in any of claims 1 to 3, wherein the first compound is present in an amount of 0.1 wt% to 5.0 wt% based on a total weight of the electrolyte for a rechargeable lithium battery.

5. The electrolyte as claimed in any of claims 1 to 4, wherein the Ag salt comprises at least one selected from AgNO₃, AgPF₆, AgFSI, AgTFSI, AgF, AgSO₃CF₃, AgBF₄, AgNO₂, AgN₃, and AgCN.

6. The electrolyte as claimed in any of claims 1 to 5, wherein the Ag salt is present in an amount of 0.1 wt% to 10.0 wt% based on a total weight of the electrolyte for a rechargeable lithium battery.

7. The electrolyte as claimed in any of claims 1 to 6,
wherein the first compound and the Ag salt are present in a weight ratio of 1:9 to 6:4.

8. The electrolyte as claimed in any of claims 1 to 7, wherein the additive further comprises a fluorinated ketone.

9. The electrolyte as claimed in claim 8, wherein the fluorinated ketone is represented by Chemical Formula 2: 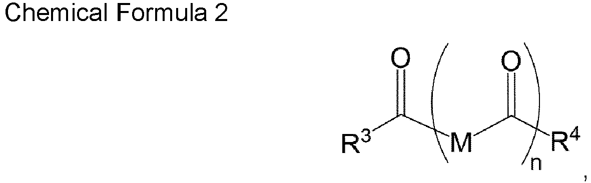 and
wherein, in Chemical Formula 2,
M is a substituted or unsubstituted C1 to C30 alkylene group, a substituted or unsubstituted C2 to C30 alkenylene group, or a substituted or unsubstituted C2 to C30 alkynylene group,
n is one of the integers from 0 to 5, and
R³ and R⁴ are each independently, a substituted or unsubstituted C1 to C30 alkyl group, a substituted or unsubstituted C2 to C30 alkenyl group, a substituted or unsubstituted C2 to C30 alkynyl group, a substituted or unsubstituted C3 to C20 cycloalkyl group, or a substituted or unsubstituted C6 to C20 aryl group, at least one of R³ or R⁴ comprising one or more fluorines (F).

10. The electrolyte as claimed in claim 8 or 9, wherein the fluorinated ketone is represented by Chemical Formula 2-1: 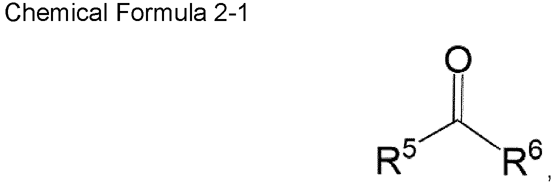 and
wherein, in Chemical Formula 2-1,
R⁵ and R⁶ are each independently a substituted or unsubstituted C1 to C30 alkyl group, and at least one of R⁵ or R⁶ comprises one or more fluorine (F).

11. The electrolyte as claimed in any of claims 8 to 10, wherein the fluorinated ketone is represented by one selected from among Chemical Formula 2-2 and Chemical Formula 2-3:

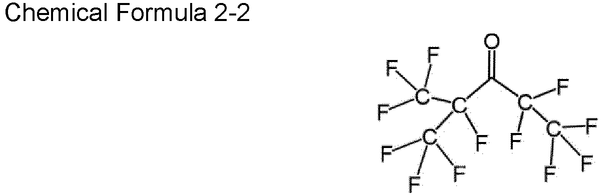

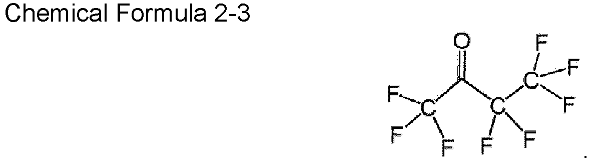

12. The electrolyte as claimed in any of claims 8 to 11, wherein the fluorinated ketone is present in an amount of about 1.0 wt% to about 10.0 wt% based on a total weight of the electrolyte for the rechargeable lithium battery.

13. The electrolyte as claimed in any of claims 1 to 12, wherein the additive is present in an amount of about 1.0 wt% to about 30.0 wt% based on a total weight of the electrolyte for the rechargeable lithium battery.

14. A rechargeable lithium battery (100), comprising
a positive electrode (114) comprising a positive electrode active material;
a negative electrode (112) comprising a negative electrode active material;
a separator (113) between the positive electrode (114) and the negative electrode (112); and
the electrolyte as claimed in claim 1.

15. The rechargeable lithium battery (100) as claimed in claim 14, wherein the negative electrode (112) has a negative electrode active material density of greater than or equal to 1.6 gram per cubic centimeter (g/cc).

## Patentansprüche

1. Elektrolyt für eine wiederaufladbare Lithiumbatterie (100), wobei der Elektrolyt Folgendes umfasst
ein nicht wässriges organisches Lösungsmittel, ein Lithiumsalz und ein Additiv, wobei das Additiv Folgendes umfasst:
eine erste Verbindung, die ausgewählt ist aus einer durch die chemische Formel 1 dargestellten Verbindung, CsPF_{6,} und einer Kombination davon; und
Ag-Salz, 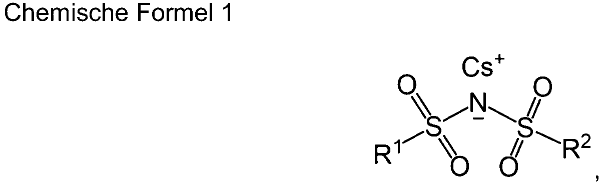
wobei in der chemischen Formel 1
R¹ und R² jeweils unabhängig eine Fluorgruppe oder eine substituierte C1- bis C10-Fluoralkylgruppe mit mindestens einem Fluor (F) sind.

2. Elektrolyt nach Anspruch 1, wobei die erste Verbindung die durch die chemische Formel 1 dargestellte Verbindung umfasst, und
wobei R¹ und R² in der chemischen Formel 1 jeweils unabhängig eine Fluorgruppe oder eine C1- bis C4-Fluoralkylgruppe sind, die mit mindestens drei Fluorgruppen substituiert ist.

3. Elektrolyt nach Anspruch 1 oder 2, wobei die erste Verbindung die durch die chemische Formel 1 dargestellte Verbindung umfasst, und
wobei die chemische Formel 1 durch eine beliebige der chemischen Formel 1-1 oder der chemischen Formel 1-2 dargestellt ist:

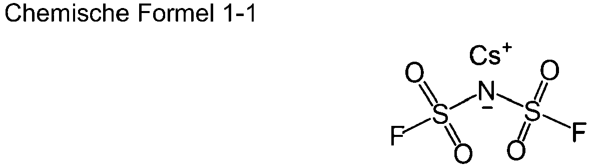

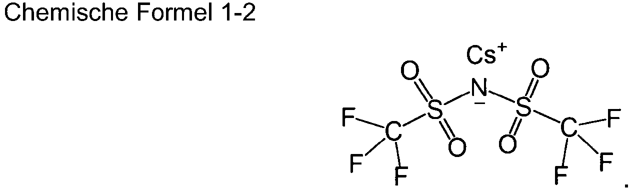

4. Elektrolyt nach einem der Ansprüche 1 bis 3, wobei die erste Verbindung in einer Menge von etwa 0,1 Gew.-% bis etwa 5,0 Gew.-% basierend auf einem Gesamtgewicht des Elektrolyten für eine wiederaufladbare Lithiumbatterie vorhanden ist.

5. Elektrolyt nach einem der Ansprüche 1 bis 4, wobei das Ag-Salz mindestens eines umfasst, das ausgewählt ist aus AgNO₃, AgPF₆, AgFSI, AgTFSI, AgF, AgSO₃CF₃, AgBF₄, AgNO₂, AgN₃ und AgCN.

6. Elektrolyt nach einem der Ansprüche 1 bis 5, wobei das Ag-Salz in einer Menge von etwa 0,1 Gew.-% bis etwa 10,0 Gew.-% basierend auf einem Gesamtgewicht des Elektrolyten für eine wiederaufladbare Lithiumbatterie vorhanden ist.

7. Elektrolyt nach einem der Ansprüche 1 bis 6,
wobei die erste Verbindung und das Ag-Salz in einem Gewichtsverhältnis von etwa 1:9 bis etwa 6:4 vorhanden sind.

8. Elektrolyt nach einem der Ansprüche 1 bis 7, wobei das Additiv ferner ein fluoriertes Keton umfasst.

9. Elektrolyt nach Anspruch 8, wobei das fluorierte Keton durch die chemische Formel 2 dargestellt ist: 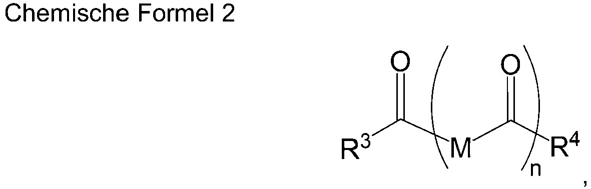 und
wobei in der chemischen Formel 2
M eine substituierte oder unsubstituierte C1- bis C30-Alkylengruppe, eine substituierte oder unsubstituierte C2- bis C30-Alkenylengruppe oder eine substituierte oder unsubstituierte C2- bis C30-Alkinylengruppe ist,
n eine der ganzen Zahlen von 0 bis 5 ist, und
R³ und R⁴ jeweils unabhängig eine substituierte oder unsubstituierte C1- bis C30-Alkylgruppe, eine substituierte oder unsubstituierte C2- bis C30-Alkenylgruppe, eine substituierte oder unsubstituierte C2- bis C30-Alkinylgruppe, eine substituierte oder unsubstituierte C3- bis C20-Cycloalkylgruppe oder eine substituierte oder unsubstituierte C6- bis C20-Arylgruppe sind, wobei mindestens eines von R³ oder R⁴ ein oder mehrere Fluoratome (F) umfasst.

10. Elektrolyt nach Anspruch 8 oder 9, wobei das fluorierte Keton durch die chemische Formel 2-1 dargestellt ist: 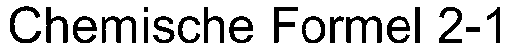 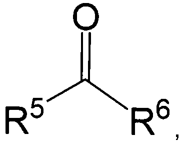 und
wobei in der chemischen Formel 2-1,
R⁵ und R⁶ jeweils unabhängig eine substituierte oder unsubstituierte C1- bis C30-Alkylgruppe sind und mindestens eines von R⁵ oder R⁶ ein oder mehrere Fluoratome (F) umfasst.

11. Elektrolyt nach einem der Ansprüche 8 bis 10, wobei das fluorierte Keton durch eine dargestellt ist, die aus der chemischen Formel 2-2 und der chemischen Formel 2-3 ausgewählt ist:

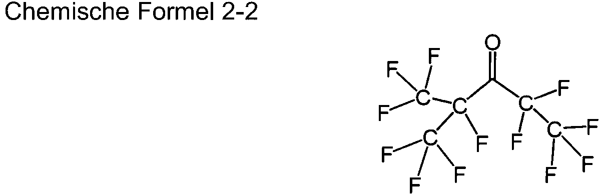

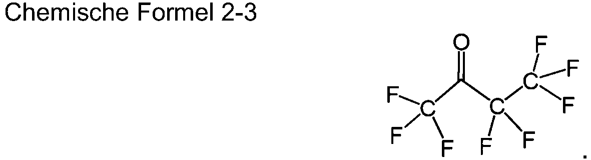

12. Elektrolyt nach einem der Ansprüche 8 bis 11, wobei das fluorierte Keton in einer Menge von etwa 1,0 Gew.-% bis etwa 10,0 Gew.-% basierend auf einem Gesamtgewicht des Elektrolyten für eine wiederaufladbare Lithiumbatterie vorhanden ist.

13. Elektrolyt nach einem der Ansprüche 1 bis 12, wobei das Additiv in einer Menge von etwa 1,0 Gew.-% bis etwa 30,0 Gew.-% basierend auf einem Gesamtgewicht des Elektrolyten für eine wiederaufladbare Lithiumbatterie vorhanden ist.

14. Wiederaufladbare Lithiumbatterie (100), umfassend
eine positive Elektrode (114), die ein Aktivmaterial für die positive Elektrode umfasst;
eine negative Elektrode (112), die ein Aktivmaterial für die negative Elektrode umfasst;
einen Trenner (113) zwischen der positiven Elektrode (114) und der negativen Elektrode (112); und
den Elektrolyten nach Anspruch 1.

15. Wiederaufladbare Lithiumbatterie (100) nach Anspruch 14, wobei die negative Elektrode (112) eine Dichte des Aktivmaterials für die negative Elektrode von mehr als oder gleich etwa 1,6 Gramm pro Kubikzentimeter (g/cc) aufweist.

## Revendications

1. Électrolyte pour une batterie rechargeable au lithium (100), l'électrolyte comportant
un solvant organique non aqueux, un sel de lithium et un additif,
dans lequel l'additif comporte :
un premier composé sélectionné parmi un composé représenté par la Formule chimique 1, CsPF₆ et une combinaison de ceux-ci ; et
un sel d'Ag, 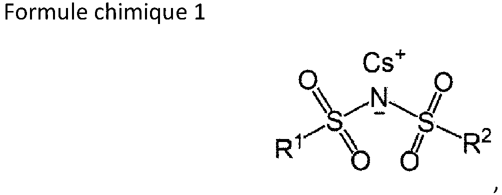
dans lequel, dans la Formule chimique 1,
R¹ et R² sont chacun indépendamment un groupe fluoro ou un groupe fluoroalkyle en C1 à C10 substitué contenant au moins un fluor (F).

2. Électrolyte selon la revendication 1, dans lequel le premier composé comporte le composé représenté par la Formule chimique 1, et
dans lequel R¹ et R² dans la Formule chimique 1 sont chacun indépendamment un groupe fluoro ou un groupe fluoroalkyle en C1 à C4 substitué par au moins trois groupes fluoro.

3. Électrolyte selon la revendication 1 ou 2, dans lequel le premier composé comporte le composé représenté par la Formule chimique 1, et
dans lequel la Formule chimique 1 est représentée par l'une quelconque de la Formule chimique 1- 1 ou de la Formule chimique 1-2 :

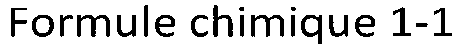

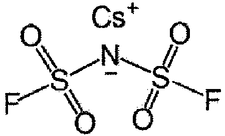

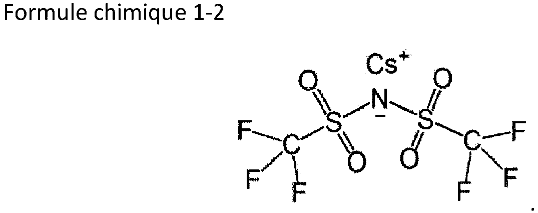

4. Électrolyte selon l'une quelconque des revendications 1 à 3, dans lequel le premier composé est présent en une quantité de 0,1% en poids à 5,0 % en poids sur la base d'un poids total de l'électrolyte pour une batterie rechargeable au lithium.

5. Électrolyte selon l'une quelconque des revendications 1 à 4, dans lequel le sel d'Ag comporte au moins l'un choisi parmi AgNO₃, AgPF₆, AgFSI, AgTFSI, AgF, AgSO₃CF₃, AgBF₄, AgNO₂, AgN₃ et AgCN.

6. Électrolyte selon l'une quelconque des revendications 1 à 5, dans lequel le sel d'Ag est présent en une quantité de 0,1 % en poids à 10,0 % en poids sur la base d'un poids total de l'électrolyte pour une batterie rechargeable au lithium.

7. Électrolyte selon l'une quelconque des revendications 1 à 6,
dans lequel le premier composé et le sel d'Ag sont présents dans un rapport pondéral de 1:9 à 6:4.

8. Électrolyte selon l'une quelconque des revendications 1 à 7, dans lequel l'additif comporte en outre une cétone fluorée.

9. Électrolyte selon la revendication 8, dans lequel la cétone fluorée est représentée par la Formule chimique 2 : 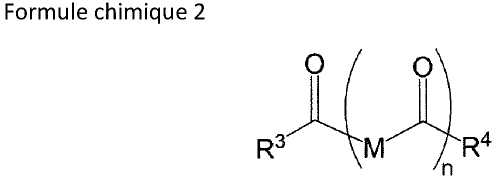 et
dans lequel, dans la Formule chimique 2,
M est un groupe alkylène en C1 à C30 substitué ou non substitué, un groupe alcénylène en C2 à C30 substitué ou non substitué, ou un groupe alcynylène en C2 à C30 substitué ou non substitué,
n est l'un des entiers de 0 à 5, et
R³ et R⁴ sont chacun indépendamment, un groupe alkyle en C1 à C30 substitué ou non substitué, un groupe alcényle en C2 à C30 substitué ou non substitué, un groupe alcynyle en C2 à C30 substitué ou non substitué, un groupe cycloalkyle en C3 à C20 substitué ou non substitué, ou un groupe aryle en C6 à C20 substitué ou non substitué, au moins l'un parmi R³ ou R⁴ comportant un ou plusieurs fluors (F).

10. Électrolyte selon la revendication 8 ou 9, dans lequel la cétone fluorée est représentée par la Formule chimique 2-1 : 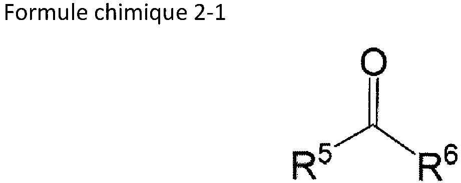 et
dans lequel, dans la Formule chimique 2-1,
R⁵ et R⁶ sont chacun indépendamment un groupe alkyle en C1 à C30 substitué ou non substitué, et au moins l'un parmi R⁵ ou R⁶ comporte un ou plusieurs fluors (F).

11. Électrolyte selon l'une quelconque des revendications 8 à 10, dans lequel la cétone fluorée est représentée par une formule sélectionnée parmi la Formule chimique 2-2 et la Formule chimique 2-3 :

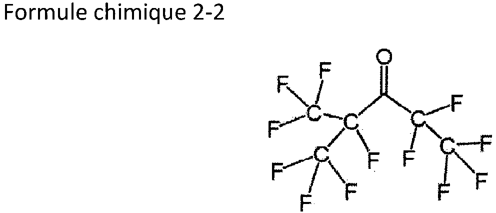

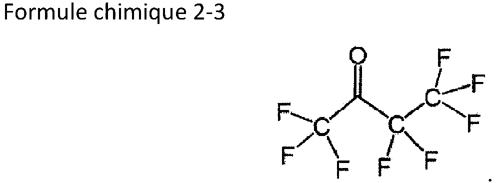

12. Électrolyte selon l'une quelconque des revendications 8 à 11, dans lequel la cétone fluorée est présente en une quantité d'environ 1,0 % en poids à environ 10,0 % en poids sur la base d'un poids total de l'électrolyte pour la batterie rechargeable au lithium.

13. Électrolyte selon l'une quelconque des revendications 1 à 12, dans lequel l'additif est présent en une quantité d'environ 1,0 % en poids à environ 30,0 % en poids sur la base d'un poids total de l'électrolyte pour la batterie rechargeable au lithium.

14. Batterie rechargeable au lithium (100), comportant :
une électrode positive (114) comportant un matériau actif d'électrode positive ;
une électrode négative (112) comportant un matériau actif d'électrode négative ;
un séparateur (113) entre l'électrode positive (114) et l'électrode négative (112) ; et
l'électrolyte selon la revendication 1.

15. Batterie rechargeable au lithium (100) selon la revendication 14, dans laquelle l'électrode négative (112) présente une densité de matériau actif d'électrode négative supérieure ou égale à 1,6 gramme par centimètre cube (g/cc).
